# EUROPEAN PATENT APPLICATION

(11) **EP 4 014 842 A1**
(43) Date of publication of application: **22.06.2022**
(21) Application number: 20215592.5
(22) Date of filing: 18.12.2020
(51) Int. Cl.: A61B 5/00

(54) **AN APPARATUS AND METHOD OF CONTROLLING A PAIN ALLEVIATING SLEEP ASSISTANCE DEVICE**

(71) Applicant: Koninklijke Philips N.V., 5656 AG Eindhoven (NL)
(72) Inventor: Bulut, Murtaza, 5656 AE Eindhoven (NL); Hilbig, Rainer, 5656 AE Eindhoven (NL); Palero, Jonathan Alambra, 5656 AE Eindhoven (NL)
(74) Representative: Philips Intellectual Property & Standards

(57) **Abstract**

According to an aspect, there is provided an apparatus (10) to control a sleep assistance device (30) for alleviating a user's pain during sleep. The apparatus (10) comprises: a receiver (11) configured to receive sensor data (14) associated with the user; a processor (12) configured to analyze the sensor data (14) to predict a pain measure of the user, and to generate a control signal (15) for the sleep assistance device (30) in accordance with the predicted pain measure; and a transmitter (13) configured to transmit the control signal (15) to the sleep assistance device (30) for alleviating pain.

## Description

### FIELD OF THE INVENTION

Embodiments of the present invention relate generally to minimizing sleep disturbances and to an apparatus, a method and a corresponding computer program for controlling a sleep assistance device for alleviating or reducing a user's pain during sleep and mitigating sleep disturbance.

### BACKGROUND OF THE INVENTION

Individuals, such as, for example, people with medical conditions and elderly people, may suffer pain or discomfort when sleeping. Such pain or discomfort may disrupt the individual's sleep by disturbing or waking the individual, leading to low quality and/or interrupted sleep.

It is therefore desirable to alleviate or reduce the pain or discomfort of an individual so that sleep disturbances and/or interruptions may be reduced or avoided and the quality of sleep of the individual may be improved.

Devices are known which may be used to relieve or reduce pain or discomfort of an individual during sleep. Such devices may, for example, cause the individual to move to a position which is less likely to result in pain or discomfort. Additionally or alternatively, such devices may, for example, apply stimulation to the individual to reduce the pain or discomfort. The optimal operation of such devices may be dependent on a number of factors.

It is therefore also desirable to control the operation of such devices, such that the effectiveness of the devices may be maximized and the pain or discomfort of the individual may be reduced.

### SUMMARY OF THE INVENTION

According to an embodiment of a first aspect, there is provided an apparatus to control a sleep assistance device for alleviating a user's pain during sleep, the apparatus comprising: a receiver configured to receive sensor data associated with the user; a processor configured to analyze the sensor data to predict a pain measure of the user, and to generate a control signal for the sleep assistance device in accordance with the predicted pain measure; and a transmitter configured to transmit the control signal to the sleep assistance device for alleviating pain.

Thus, the sleep assistance device may be controlled in accordance with the sensor data and the predicted pain measure of the user. The sleep assistance device may therefore be controlled in a manner that is suited to the condition of the user and the effectiveness of the sleep assistance device may be maximized. By maximizing the effectiveness of the sleep assistance device, the pain relief provided to the user may also be maximized. The pain of the user may therefore be relieved or reduced and sleep disturbances or interruptions may be avoided. That is, the sleep assistance device may be controlled in accordance with the predicted pain measure of the user so that the stimulation of the sleep assistance device may be appropriately delivered to the user. Prediction of the pain measure and the associated control of a sleep assistance device may allow pain relief to be delivered to the user prior to the user being disturbed or woken. Embodiments of aspects may therefore be considered as proactive and preventative, rather than reactionary. The effectiveness of the sleep assistance device may therefore be increased and disturbance to the user's sleep may be minimized.

The sleep assistance device may be any device for assisting with the sleep of the user so as to minimize sleep disturbance or interruptions. A sleep assistance device may assist the sleep of the user by reducing or alleviating pain or discomfort of the user, thereby reducing the likelihood of the user waking or being disturbed during sleep. The sleep assistance device may be a pain relief device or a position change device. Additionally or alternatively, the sleep assistance device may be a sound generation device, a temperature changing device, an air quality and air circulation changing device, and/or a medication providing device. The sleep assistance device may therefore be any device that is capable of influencing the user during sleep. For example, the sleep assistance device may also be a smart watch which may use the vibration properties and/or audio of the watch to trigger the user to change position. Thus, a pain relief device may be activated and controlled in accordance with the predicted pain measure of the user.

Alleviating pain may also be referred to as mitigating, relieving or reducing pain. The sleep assistance device may be wearable or not wearable. That is, they may be attached to the body of the user or not attached to the user, but in the sleeping environment, such as integrated in bed, or around the bed in various parts of the room.

The pain measure may relate to a predicted pain or discomfort level of the user. Pain may be categorized as a feeling of discomfort or uncomfortable sensations of the user, related to activation of the nervous system. Any type or cause of pain may be considered. Pain has a well-established meaning in medical and healthcare fields. Pain may also relate to interpreted pain of the user, which may differ depending on a number of factors. Pain may be associated with a health condition or an injury of the user.

The pain measure may be predicted and monitored so that the pain or discomfort may be appropriately managed by the use of the sleep assistance device. The pain measure may be predicted and monitored over time to determine a rate of change of the pain measure. The control signal may be generated in accordance with the rate of change of the pain measure. For example, if the pain measure is increasing, the sleep assistance device may be controlled accordingly (for example, activated and/or the setting/operation mode adjusted), so as to reduce the pain measure. By predicting the pain measure, the apparatus may activate and control the sleep assistance device so as to reduce the pain felt by the user.

By predicting the pain and controlling the sleep assistance device in accordance with the predicted pain, the device may cause the user's pain to be relived prior to the user being disturbed. Sleep disturbances may therefore be avoided and quality of sleep may be improved. The control signal may also be referred to as an instruction signal or an activation signal. The control signal may be a signal for transmitting information to the sleep assistance device which causes the device to operate and/or alter its operation.

The pain measure may be a predicted pain level and the pain level may be compared with one or more predetermined thresholds. For example, a control signal to activate the sleep assistance device may be generated in response to the predicted pain level exceeding a first predetermined threshold. Similarly, a control signal to change the setting of the sleep assistance device (for example, to increase the intensity level) may be generated in response to the predicted pain level exceeding a second predetermined threshold which is larger than the first predetermined threshold. Accordingly, the sleep assistance device may be controlled in accordance with the predicted pain measure. The intensity or level of stimulation provided by the sleep assistance device may be increased as the predicted pain level increases. If the sensor data is motion data that indicates an amount of movement of the user, a greater pain measure may be predicted in response to a greater amount of movement of the user being detected. The predicted pain measure may also be an input to a different function or a machine learning algorithm, and the control signal may be generated in accordance with these results.

The sensor data may be received from a sensor device or a number of sensor devices associated with the user and configured to acquire sensor data relating to the user. For example, the sensor device(s) may measure physiological or environment parameters associated with the user and the user's sleep, i.e. while the user is sleeping. The sensor device may also be referred to as a sleep monitoring device. The measured parameters may be parameters that are indicative of the sleep of the user, or may be used to derive information indicative of the sleep of the user. For example, the sensor may be an EEG device that acquires EEG data to monitor the brain activity of the user. The sensor data may be analyzed to predict a pain measure of the user. For example, the EEG data may indicate that the user is feeling pain or discomfort. Additionally or alternatively, the sensor may be a camera configured to acquire images or a sequence of images (for example, video) of the user (image/video data). The images may then be analyzed (for example, using facial recognition technology) to predict a pain measure of the user. For example, the image data may be analyzed to determine whether the user's face is displaying signs of pain, discomfort or anguish.

The sensor device may be provided separately from or together with the apparatus. Multiple sensor devices may be provided and the apparatus may receive sensor data from each of the devices. If sensor data is received from multiple sensor devices, the apparatus may analyze all or only some of the received sensor data. The apparatus may also filter the sensor data and/or may apply an order of preference or weighting to the sensor data, such that sensor data received from one sensor device may take priority over or may have a higher weighting than sensor data received from another sensor device.

The apparatus may be communicably coupled with the sleep assistance device and the transmitter may transmit the control signal to the sleep assistance device by any suitable means. For example, the apparatus may be wirelessly connected to the sleep assistance device and the transmitter may transmit the control signal to the sleep assistance device using an appropriate wireless communication means, such as, for example, Bluetooth, near-field communication, Wi-Fi, etc. Alternatively, the apparatus may be connected to the sleep assistance device via a wired communication link and the transmitter may transmit the control signal to the sleep assistance device using the wired link. The apparatus and the sleep assistance device may be provided as a single device and the transmitter may transmit the control signal to the sleep assistance device via a communication bus. Multiple sleep assistance devices may be provided and the transmitter may transmit control signals to one or more of the plurality of sleep assistance devices. The same or different communication means may be used for each sleep assistance device. The sleep assistance device may provide information and data to the apparatus and may use the same communication link via which it receives the control signal.

The processor may compare the sensor data with known data. The processor may predict the pain measure of the user based on the sensor data and the known data. That is, a comparison of the sensor data and the known data may be performed and the comparison may be used in the prediction of the pain measure. For example, the known data may indicate the expected ranges of the measured parameters (such as, for example, EEG activity, heart rate) and the measured parameters included in the sensor data may be compared with the expected ranges to predict the pain measure of the user.

An amount of variance between the sensor data and the known data may therefore be used in the prediction of the pain measure. A higher variance between the sensor data and the known data may result in the processor predicting a higher pain measure. For example, if the known data relates to expected measurements when the user is in pain, then a high pain measure may be predicted if the known data and the sensor data are similar and the variance is small. Conversely, if the known data relates to expected measurements when the user is not in pain, then a high pain measure may be predicted if the variance between the sensor data and the known data is large, i.e. above a predetermined threshold.

The known data may be provided from readily available medical and scientific sources and may be stored in a memory of the apparatus and/or may be received from another device, such as, for example, a network or cloud server. For example, the known data may be expected parameters (for example, heart rate and heart rate variability) for an individual that is similar to the user (for example, the same age group and/or gender as the user). If the sensor data is EEG data, the known data may be the expected EEG readings for such an individual which are established in the art.

Alternatively or additionally, the known data may be data that is acquired from the user. For example, the user may be monitored over time to acquire expected readings for when the user does and does not feel pain or discomfort. The acquired sensor data may therefore be compared with the user's 'normal' readings and the pain measure predicted accordingly.

The use of the expected data may therefore allow for the accuracy of the predicted pain measure to be improved. Furthermore, sensor data may be monitored over time to establish 'normal' expected values for the user, which may be used as the known data, thereby tailoring the pain measure prediction to the user.

The processor may analyze the sensor data to determine a stage of sleep of the user. The processor may predict the pain measure of the user based on the sensor data and the stage of sleep of the user. That is, the sensor data may be used to determine the stage of sleep of the user and the stage of sleep of the user may be used in the prediction of the pain measure.

Accordingly, the stage of sleep of the user may also be used in the generation of the control signal, such that the sleep assistance device is controlled in accordance with the stage of sleep of the user. The prediction of the pain measure and the control of the sleep assistance device may therefore be improved since the user's perception of pain may vary depending on the sleep stage and the optimum settings of the sleep assistance device may also vary depending on the sleep stage. For example, if the user is in a light stage of sleep, the predicted pain measure may be higher than a pain measure predicted during a deep stage of sleep. Furthermore, an intense setting of the sleep assistance device that provides a large amount of stimulation to the user may disturb the user if they are in a stage of light sleep and so, if it is determined that the user is in stage of light sleep, the control signal may be generated to limit the intensity level of the sleep assistance device.

Expected physiological parameters (such as, for example, EEG data, heart rate, skin temperature, movement, etc.) for the various stages of sleep for a person are known in the art. Thus, the processor may determine the stage of sleep by comparing the sensor data with the expected data and determining that the user is in the stage of sleep for which the sensor data most closely matches the expected data. Furthermore, the sleep stages are well defined and cyclical. The stage of sleep may therefore be determined based on the duration of sleep and/or a previous sleep stage.

The processor may analyze the sensor data to predict a sleep disturbance event in a predetermined time period. The transmitter may generate the control signal for the sleep assistance device in accordance with the predicted sleep disturbance event so as to minimize the possibility of occurrence of the predicted sleep disturbance event. That is, a sleep disturbance event may be predicted from the sensor data before the event occurs. The sleep assistance device may then be controlled in accordance with the predicted event so as to reduce the chance of the sleep disturbance event occurring. By predicting the sleep disturbance event ahead of time and taking appropriate action by controlling the sleep assistance device, the event may be avoided and the user's sleep may be improved. That is, prediction of a sleep disturbance event and the associated control of a sleep assistance device may allow pain relief to be delivered to the user prior to the user being disturbed or woken. Embodiments of aspects may therefore be considered as proactive and preventative, rather than reactionary. The effectiveness of the sleep assistance device may therefore be increased and disturbance to the user's sleep may be minimized

The sleep disturbance event may be related to the predicted pain measure. For example, a disturbance event may be predicted in response to the predicted pain measure exceeding a predetermined threshold, and/or the rate of change of the predicted pain measure exceeding a predetermined threshold. That is, for example, a sleep disturbance event may be predicted if it is determined that the user's pain level is rising. The sleep assistance device may be activated or the setting of the device may be adjusted so as to reduce the pain measure of the user and prevent sleep disturbance.

A sleep disturbance event may be any event that interrupts the normal sleep cycle of the user and/or wakes the user. Such events are unwanted and so it is desirable to prevent these events from happening. Prediction of such an event in a subsequent time period may enable the event to be avoided. The time period may be, for example, 3 to 5 minutes, 5 to 10 minutes, 5 to 15 minutes, etc. That is, from the received sensor data, the processor may predict whether a sleep disturbance event will occur in a subsequent period, such as, for example, the next 5 to 10 minutes. In response to a sleep disturbance event being predicted, the processor may generate a control signal that, for example, activates a sleep assistance device.

The processor may analyze the sensor data to determine a stage of sleep of the user. The processor may compare the sensor data with expected data for the stage of sleep. The processor may predict the sleep disturbance event based on the comparison of the sensor data and the expected data. That is, the stage of sleep of the user may be determined and a sleep disturbance event may be predicted in accordance with the determined stage of sleep of the user and the expected data for that stage of sleep. Thus, the accuracy of the sleep disturbance event prediction may be improved. Furthermore, the sleep disturbance event may be predicted in advance so that preventative action may be taken to avoid the sleep disturbance event.

Expected physiological parameters (such as, for example, EEG data, heart rate, movement) for the various stages of sleep for a person are known in the art. Thus, by comparing the sensor data with expected data for a sleep stage, deviations from the expected data may be detected, and such deviations may be indicative of an approaching sleep disturbance event. For example, a sleep disturbance event may be predicted in response to the sensor data deviating from the expected data by a predetermined amount and/or for a predetermined amount of time. A sleep disturbance event may therefore be predicted.

The expected data may be provided from readily available medical and scientific sources and may be stored in a memory of the apparatus and/or may be received from another device, such as, for example, a network or cloud server. For example, the expected data may be expected parameters (for example, heart rate and heart rate variability) for an individual that is similar to the user (for example, the same age group and/or gender as the user). If the sensor data is EEG data, the expected data may be the expected EEG readings for such an individual which are established in the art.

The processor may store the sensor data in a memory associated with the apparatus. The processor may train a classifier using the sensor data stored in the memory. The processor may predict the sleep disturbance event in accordance with a likelihood score of the classifier. That is, a classifier may be trained using sensor data that is acquired over time such that received sensor data may be classified by the classifier. For example, the classifier may be a binary classifier that predicts a sleep disturbance event (yes or no). For example, a sleep disturbance event may be determined if a predetermined classifier threshold is exceeded by the received sensor data. Thus, previous sensor data relating to the user may be used to predict future sleep disturbance events, which may make the prediction more accurate for the user.

During a sleep onset period prior to the user falling asleep, the receiver may receive initialization information. The initialization information may comprise data indicative of one or more of: a position of the user, and physiological signals of the user. The processor may predict the pain measure of the user based on the sensor data and the initialization information. In other words, prior to the user falling asleep, the apparatus may receive sensor data relating to this period, which is initialization data, and this data may be used in the prediction of the pain measure.

Prior to falling asleep, it may be assumed that the user will consciously lie in a position that does not cause any (or minimizes) pain and/or discomfort. Thus, the data acquired during this period may be used as reference data to improve the accuracy of the pain measure prediction. For example, if the data relates to a position of the user, this data may be used as a reference point for comparing sensor data acquired during sleep. If the user is in the same position during sleep as they are in during the sleep onset stage, then a low pain measure may be predicted. The user's pain/discomfort levels may fluctuate day-to-day and so the initialization information may be acquired daily. Alternatively, it may be acquired weekly or monthly for example. Using the initialization information acquired during the sleep onset period, the predictive algorithm and device activation settings may be re-tuned when necessary. The initialization information may similarly be taken into account when predicting a sleep disturbance event.

Additionally or alternatively, the initialization information may be used in the generation of the control signal. That is, the settings of the sleep assistance device may be adjusted using the initialization information. For example, if a high pain measure or sleep disturbance event is predicted, the apparatus may generate a control signal that causes the sleep assistance device to move the user to a position that reflects the position assumed by the user during the sleep onset period.

The initialization data may be received from one or more sensor devices associated with the user. The sleep onset period may be a period between the user beginning to try to fall asleep and the user falling asleep. The physiological signals of the user may comprise heart rate, heart rate variability, respiration rate, blood pressure, skin temperature, EEG, skin conductance, movement, sound and/or respiration depth. The position of the user and the physiological signals of the user may correspond to the sensor data.

The receiver may receive user data. The processor may predict the pain measure of the user based on the sensor data and the user data. The user data may comprise: one or more of: the age of the user; medical history of the user; medication prescribed to the user; medication taken by the user; a medical diagnosis of the user; lifestyle information of the user; sleeping preferences of the user; sleep environment of the user; a location of the user; the gender of the user; the ethnicity of the user; physical activity of the user; information on the user's preferred operating settings of the sleep assistance device; subjective input of the user; and an occupation of the user. Thus, information on the user may be used in the pain measure prediction and the accuracy of the prediction may be improved.

The information on the user may affect the pain perceived by the user and so it may be beneficial to take this information into account when predicting the pain measure of the user. For example, if it is known that the user has an injury to his/her right arm, then the pain measure may be predicted to be high if the user is lying on his/her right arm. Similarly, if it is known that the user takes pain relief medicine that lasts for four hours immediately before bed, then the pain measure may be predicted to be higher after the medicine has worn off compared with before. A sleep assistance device may therefore be activated when the medicine wears off so as to compensate for the lack of pain relief medicine in the user's system. The user information may similarly be taken into account when predicting a sleep disturbance event.

The user information may be received from a user device, such as, for example, a smart phone of the user. Additionally or alternatively, the user information may be input to the apparatus and may be stored in a memory of the apparatus. The user information may also be retrieved from another memory device, such as, for example, a server, which may be a cloud server. For example, a medical professional may provide user information to a server (such as, for example, information on medication prescribed to the user) and the apparatus may retrieve the user information. Different types of user information may be received from different devices. A subjective input of the user may relate to a sleep diary kept by the user. Many sleep applications facilitate keeping sleep diaries and information input to these may be utilized in the prediction of the pain measure. The entries in the sleep diaries may be automatically processed using Natural language processing to learn the user's perception of sleep quality as well as the pain perception.

The sleep assistance device may be one of a plurality of sleep assistance devices. The processor may select the sleep assistance device from among the plurality of sleep assistance devices by analyzing the sensor data. That is, multiple sleep assistance devices may be available and the apparatus may select the one or more devices from the available devices. The selected device(s) may, for example, be determined to be the most appropriate device(s) that will provide the most effective relief to the user in the circumstances. The effectiveness of the sleep assistance devices may therefore be maximized. The sleep assistance device may similarly or additionally be selected in accordance with a predicted sleep disturbance event.

For example, a pain relief device and a position change device may be available. If, by analysing the sensor data, the apparatus determines that the user is in a light stage of sleep, then the apparatus may select the position change device over the pain relief device since the pain relief device may wake the user when they are in a stage of light sleep. Multiple sleep assistance devices may be used at the same time such that they are controlled in cooperation with each other. The apparatus may therefore select multiple sleep assistance devices from the available devices by analyzing the sensor data to determine the most appropriate devices to select.

The processor may determine a setting instruction for the sleep assistance device in accordance with the predicted pain measure of the user. The control signal may comprise the setting instruction for the sleep assistance device. That is, the control signal comprises instructions for the sleep assistance device relative to the predicted pain measure. The use of the sleep assistance device may therefore be optimized. The setting information may similarly or additionally be determined in accordance with a predicted sleep disturbance event.

For example, the control signal may comprise an instruction for the sleep assistance device to be active for a predetermined period of time. If the sleep assistance device has different settings or modes, the control signal may also comprise an instruction for the setting or mode of the sleep assistance device. The setting or mode may be instructed in view of the predicted pain measure and/or sleep disturbance event. For example, if the pain measure is predicted to be high, then a high setting of a pain relief device may be instructed.

If multiple sleep assistance devices are to be used, then the setting instruction for one device may be determined in accordance with the setting instructions for another device. For example, a position change device may be instructed to cause the user to move to a position at which a pain relief device may be applied.

The receiver may receive feedback data from the sleep assistance device. The feedback data may comprise one or more of: present setting instruction of the sleep assistance device; a duration of operation of the sleep assistance device; status information of the sleep assistance device; and operating restrictions of the sleep assistance device. The processor may generate the control signal in accordance with the feedback data. Thus, feedback information from the sleep assistance device may be received by the apparatus and may be taken into account when generating the control signal. The sleep assistance device may therefore be controlled in view of its current settings, current operation, duration of operation and/or limitations of the sleep assistance device. The control of the sleep assistance device may therefore be improved. The feedback data may also be used in the prediction of the pain measure and/or the prediction of the sleep disturbance event. Daily sleep comfort or discomfort measure may be calculated based on the sleep duration and device usage duration. This measure may be used to monitor sleep quality, or user pain level, or user discomfort level during sleep. Many wearable trackers generate a sleep quality indicator and various objective sleep metrics may be calculated based on the sensor data, the time of device usage and the user's response to the device usage. This information may be used in the prediction of the pain measure, the generation of the control signal and/or the prediction of a sleep disturbance event.

For example, the sleep assistance device may have a maximum operating time of five minutes and may have been operating for four and a half minutes. The apparatus may therefore instruct the sleep assistance device to switch off. The apparatus may also instruct another device to switch on to replace the device. The status information may include one or more of: battery level of the device; a temperature of the device; and a position of the device relative to the bed and/or the user. The position of the user may refer to the position of the user in relation to the bed and may also be considered as an orientation. It may also refer to one or more of: the center of mass location, position/orientation of legs with respect to head, position/orientation of arms with respect to head, user laying on back, on stomach, on left side, on right side, etc.

The processor may store the feedback information in a memory associated with the apparatus in accordance with the sensor data received with the feedback information. The processor may analyze the stored feedback information and sensor data to determine an optimum mode of operation of the sleep assistance device for the user. The processor may generate the control signal in accordance with the optimum mode of operation of the sleep assistance device. Thus, the apparatus may use the previously acquired data to learn the optimum settings of the sleep assistance device. The feedback information may be stored in accordance with corresponding sensor data, the corresponding predicted pain measure and/or a corresponding predicted sleep disturbance event.

For example, the feedback information indicating the settings of the sleep assistance device that corresponds to a pain measure that is low may be considered to be an optimum setting of the sleep assistance device since the pain level of the user was predicted to be low when these settings were applied. By storing such data over time and using it to optimize the generation of the control signal, the effectiveness of the sleep assistance device may be improved and the efficiency with which relief is provided to the user may be improved. Machine learning may be used to determine the optimum mode of operation from the stored data. The memory may be a memory of the apparatus or another memory device communicably connected to the apparatus, such as, for example, a networked or cloud server.

The sensor data may be one or more of: electroencephalogram, EEG, data; movement data; image data; respiration data; heart data; electrodermal data; blood pressure data; audio data; and temperature data. The sensor data may therefore be any data associated with the user that may be relevant for monitoring the user's sleep and/or perceived pain.

The sensor data may be received from a single sensor device or multiple sensor devices. For example, the EEG, data may be received from an EEG sensor; the movement data may be received from an accelerometer; the image data may be received from a camera; the respiration data may be received from a respiration sensor; the heart data may be received from a heart rate sensor; the electrodermal data may be received from an electrodermal sensor; the blood pressure data received from a blood pressure sensor; the audio data may be received from a microphone; and the temperature data may be received from a thermometer. Alternatively or additionally, multiple data types may be acquired from a single sensor or combination of sensors. For example, the respiration data, the movement data and the heart data may be received from an accelerometer. The heart data may comprise heart rate data and/or heart rate variability (HRV) data.

According to an embodiment of a second aspect, there is provided a method of controlling a sleep assistance device for alleviating a user's pain during sleep, the method comprising: receiving sensor data associated with the user; analyzing the sensor data to predict a pain measure of the user; generating a control signal for the sleep assistance device in accordance with the predicted pain measure; and transmitting the control signal to the sleep assistance device for alleviating pain.

According to an embodiment of a third aspect, there is provided a computer program which when executed carries out a computer-implemented method of controlling a sleep assistance device for alleviating a user's pain during sleep, the method comprising: receiving sensor data associated with the user; analyzing the sensor data to predict a pain measure of the user; generating a control signal for the sleep assistance device in accordance with the predicted pain measure; and transmitting the control signal to the sleep assistance device for alleviating pain.

Features and sub-features of the method and computer program aspects may be applied to the apparatus aspects and vice versa.

According to an embodiment of a fourth aspect of the invention there is provided a non-transitory computer-readable medium storing a computer program as described above.

According to an embodiment of a fifth aspect of the invention there is provided a system comprising: a sensor device for acquiring sensor data associated with a user during sleep; a sleep assistance device for alleviating pain of the user; and an apparatus comprising: a receiver configured to receive the sensor data from the sensor device; a processor configured to analyze the sensor data to predict a pain measure of the user, and to generate a control signal for the sleep assistance device in accordance with the predicted pain measure; and a transmitter configured to transmit the control signal to the sleep assistance device.

An apparatus or computer program according to preferred embodiments of the present invention may comprise any combination of the method aspects. Methods or computer programs according to further embodiments may be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software.

Thus according to one aspect there is provided a program which, when loaded onto at least one computer configures the computer to become the apparatus according to any of the preceding apparatus definitions or any combination thereof.

According to a further aspect there is provided a program which when loaded onto the at least one computer configures the at least one computer to carry out the method steps according to any of the preceding method definitions or any combination thereof.

In general the computer may comprise the elements listed as being configured or arranged to provide the functions defined. For example this computer may include memory, processing, and a network interface. In other words, according to an embodiment of an aspect, there may be provided an apparatus for alleviating a user's pain during sleep, the apparatus comprising one or more processors and a memory, the one or more processors configured to perform the functions defined.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention may be performed in a different order and still achieve desirable results.

Elements of the invention have been described using the terms "receiver", "processor", "transmitter", "memory" etc. The skilled person will appreciate that such terms and their equivalents may refer to parts of the system that are spatially separate but combine to serve the functions defined. Equally, the same physical parts of the system may provide two or more of the functions defined.

For example, separately defined means may be implemented using the same memory and/or processor as appropriate.

### BRIEF DESCRIPTION OF THE DRAWINGS

Exemplary embodiments will now be described, by way of example only, with reference to the following drawings, in which:
Fig. 1 is a block diagram of main apparatus components according to a general embodiment of an aspect of the invention;
Fig. 2 is a flowchart of a method according to a general embodiment of an aspect of the invention;
Fig. 3 is a block diagram of a system according to an embodiment of an aspect of the invention;
Fig. 4 is a flow diagram of a control method according to an embodiment of an aspect of the invention;
Fig. 5 is a graph showing the expected physiological readings of an individual in the different stages of sleep;
Fig. 6 is a graph of the stages of sleep against time in a standard sleep cycle; and
Fig. 7 is a hardware diagram illustrating hardware that may be used to implement invention embodiments.

### DETAILED DESCRIPTION OF THE EMBODIMENTS

Embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein may be used in the practice or testing of the embodiments.

Embodiments of aspects may provide an apparatus, method, computer program and system of controlling a sleep assistance device for alleviating a user's pain during sleep.

As discussed above, many individuals, such as elderly people or people with medical conditions, suffer from chronic pain which often results in increased sleep fragmentation or disturbance, thereby directly influencing their sleep quality. It is therefore desirable to reduce an individual's pain so as to minimize sleep disturbances and improve sleep quality. In other words, it is desirable to avoid sleep disturbance events, such as those caused by pain, so that a person's sleep is not interrupted.

A person's perception of pain may be dependent on their sleep stage (such as, for example, their electroencephalogram, EEG, activity), their sleeping position and/or their movements. Pain in relation to sleep disturbance therefore varies depending on the stage of sleep. That is, a level of pain that may cause a disturbance to the person when they are in a light sleep state may not be sufficient to disturb the person when they are in a deep sleep state. Sleep assistance devices may be used to alleviate pain and reduce sleep disturbances. Optimum settings of sleep assistance devices may vary depending on the stage of sleep of the user. For example, a high or intense setting of a sleep assistance device may actually disturb the user when they are in a light sleep state. It is therefore desirable to control these devices in accordance with the stage of sleep of the user so that pain may be effectively reduced or managed and sleep disturbance events may be avoided or minimized.

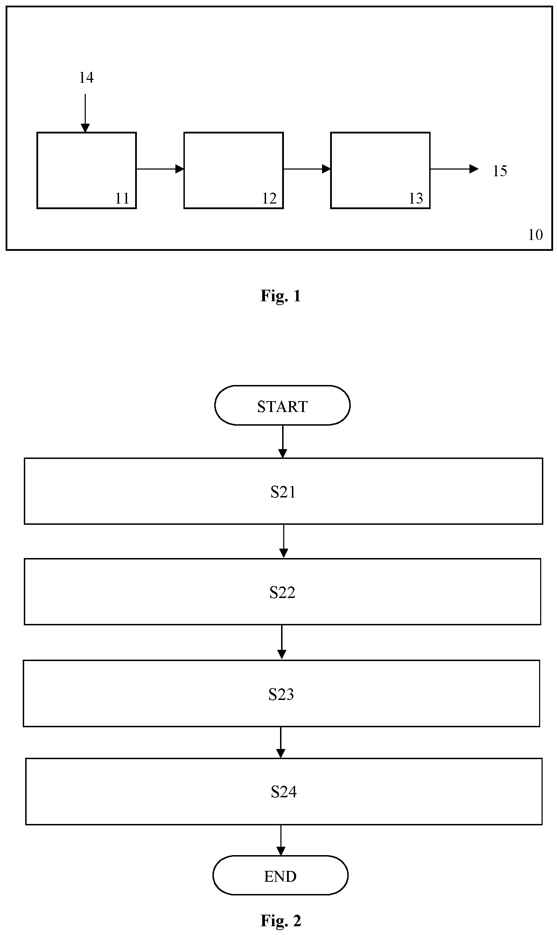

Fig. 1 shows a block diagram of information flow into main apparatus components in apparatus 10. The apparatus 10 comprises a receiver 11, a processor 12 and a transmitter 13. Sensor data 14 associated with a user is received by the receiver 11. The sensor data 14 may therefore be data relating to the user and the user's condition. The processor 12 analyzes the received sensor data 14 to predict a pain measure of the user. In accordance with the predicted pain measure, the processor 12 generates a control signal 15 for a sleep assistance device, which is a device for alleviating or reducing pain or discomfort of the user. The control signal 15 is transmitted by the transmitter 13 to the sleep assistance device, so that the sleep assistance device may be controlled in accordance with the predicted pain measure of the user.

The apparatus may be a suitable processing device that is capable of being communicably connected to other devices. The device may, for example, be a smart phone associated with the user, or may be a smart home device, which is able to store and process data from multiple sleep monitoring devices. Additionally, the apparatus may be provided as part of a cloud computing network such that some of the processing is performed in the cloud. The apparatus may also be one of the monitoring/sensor devices that measures the sensor data. In this case, the device may still be connected to other sensor devices and/or the cloud. That is, the sensor device may receive data from the other devices, perform the analysis of the data, generate control signals for the sleep assistance device(s) and transmit the control signals to the devices. Alternatively, the apparatus may be a sleep assistance device which is connected to and receives data from the other devices.

Fig. 2 shows a flow chart representing the method according to a general embodiment of an aspect of the invention. Firstly, in step S21, sensor data is received. The sensor data is associated with a user, such that the received sensor data may relate to the user or the user's condition. The received sensor data is then analyzed to predict a pain measure of the user at step S22. Next, at step 23, the predicted pain measure is used to generate a control signal for a sleep assistance device. That is, a control signal for the sleep assistance device is generated in accordance with the predicted pain measure of the user, such that the sleep assistance device may be controlled based on the predicted pain of the user. Finally, in step S24, the generated control signal is transmitted to the sleep assistance device, which is a device for alleviating pain or discomfort of the user.

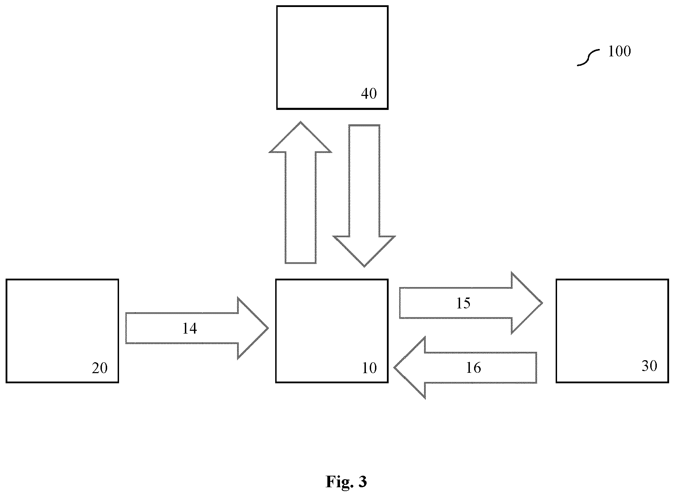

Fig. 3 shows a block diagram of a system according to an embodiment of an aspect of the invention. The system 100 comprises an apparatus 10, a sensor device 20, a sleep assistance device 30 and a memory 40. The sensor device 20 acquires sensor data 14 associated with a user during sleep and the sleep assistance device 30 is for alleviating pain or discomfort of the user. The apparatus 10 receives sensor data 14 from the sensor device 20, analyzes the sensor data 14 to predict a pain measure of the user, generates a control signal 15 for the sleep assistance device 30 in accordance with the predicted pain measure and transmits the control signal 15 to the sleep assistance device 30. The sleep assistance device 30 may send feedback data 16 to the apparatus 10. The apparatus 10 may send data to and receive data from the memory 40.

Embodiments of the present invention may therefore enable a sleep assistance device to be controlled in accordance with sensor data and a predicted pain measure of the user. The sleep assistance device may therefore be controlled in a manner that is suited to the condition of the user and the effectiveness of the sleep assistance device may be maximized. By maximizing the effectiveness of the sleep assistance device, the pain relief provided to the user may also be maximized. The pain of the user may therefore be relieved or reduced and sleep disturbances or interruptions may be avoided. That is, the sleep assistance device may be controlled in accordance with the predicted pain measure of the user so that the stimulation of the sleep assistance device may be appropriately delivered to the user. The effectiveness of the sleep assistance device may therefore be increased and disturbance to the user's sleep may be minimized since pain may be relived prior to it disturbing or waking the user.

Embodiments of the present invention may therefore prevent disturbances to the user's sleep, by predicting such disturbances and taking necessary measures to eliminate or minimize the factors (either directly, or indirectly) that contribute to the sleep disturbance. It may be considered that two types of predictions are provided:
(i) Sleep disturbance (for example, wake up) prediction
(ii) Increased pain (perception) prediction

The sleep disturbance prediction may be direct and indirect.
- Direct: the sleep disturbance may be estimated directly from EEG and movement data. This may be achieved, for example, by training machine learning algorithms.
- Indirect: First pain increase (pain measure) may be estimated (for example, from EEG, movement, timing of medications, etc.), and based on the pain estimate and EEG and movement data, sleep disturbance may be estimated.

Pain detection and localization may be achieved by training a neural network classifier from brain signals (EEG data). According to an embodiment of an aspect, EEG data is used to train a neural network classifier. Additionally, motion data may be used if available. Other data may also be used such as, for example, medication intake information (time and type of medication). If pain medications are used, accurate predictions of the time periods when pain is expected may be determined using the pharmacokinetics and pharmacodynamics of the corresponding medications, well before physiological signals start to change. That is, if the time and type of medication is known, the time when the effect of the medication is likely to wear off may be predicted and taken into account in the pain measure and/or sleep disturbance event prediction.

Other data that may be used in the pain prediction and sleep disturbance prediction may include information on the user and their sleeping environment. For example, the data may include information on: the age of the user; the medical history of the user; medication prescribed to the user; a medical diagnosis of the user; lifestyle information of the user; sleeping preferences of the user; sleep environment of the user; a location of the user; the gender of the user; the ethnicity of the user; physical activity of the user; and/or an occupation of the user.

In addition to the EEG and motion data, other physiological signals may also be used for predicting wake-up and pain increase. Physiological signals such as respiration (rate and depth), heart signals (heart rate and heart rate variability), electrodermal activity (skin conductance, and skin temperature) and sound signals (sound of breathing, sound of motion, coughing, and speech-like signals in sleep) may be used. All of these may be monitored in real-time, or close to real-time.

The sensor data may therefore be acquired from any monitoring device or sensor which is capable of detecting/measuring appropriate metrics of the user that provide information on the user's sleep. The sensor data may be; electroencephalogram, EEG, data; movement data; image data; respiration data; heart data; electrodermal data; blood pressure data; audio data; and temperature data. If the sensor data is image data, an image (or sequence of images) of the user's face may be analyzed to detect facial expression changes that indicate that the user is in pain.

There is well-established literature concerning different sleep stages and the corresponding EEG signals features during these sleep stages. According to an embodiment of an aspect, one way to predict the disruption of sleep-patterns is by detecting more than expected variations in the EEG signals. For instance, in stage 1 sleep EEG signals are expected to be mainly in F1 range (frequency) and with A1 (amplitude). If the current monitoring shows larger variations than these expected ranges (F1 and A1), this can be considered as a potential trigger for sleep disturbance. Consequently, the system can be activated to prevent further deviations, and bring the signals to the levels expected of them for a given sleep stage. In other words, this can be considered as a feedback system, where the effect and adaptation of sleep assistance device(s) (for example, position change and/or pain relief) is guided by the changes in the EEG signals.

The stages of sleep may be categorized as follows:
- Wakefulness: High frequency, low-voltage, which is usually beta-band corresponding to 13-24 Hz.
- Falling asleep and Stage 1: Alpha band (8-12Hz), emerging theta-wave (4-7Hz). Breathing and heart rate decline.
- Stage 2: Sleep spindles.
- Stage 3 and Stage 4: Delta waves (4Hz or less).
- REM (Stage 5): EEG somewhat similar to wakefulness, irregular breathing and heart rate, rapid eye movements. Although EEG patterns are somewhat similar to wakefulness, rapid eye-movements and time of occurrence (i.e. preceding sleep stages) can be used to differentiate this from wakefulness. It is difficult to wake a person during this stage.

Another implementation method for prediction may be to have personalized definitions of wakefulness, sleep-disruption and pain-perception. In other words, user data may be used define these stages. Instead of using general definitions of these, having a personalized definition may improve the prediction accuracy. For example, if person A is known to mostly wake-up during stage 1, this sleep stage can also be defined as wake-up period, and since the sleep stages have a well-defined pattern, pain reduction methods can be activated during the REM-stage. Alternatively or additionally, if it is known that the likelihood for the user waking up is much higher when they are in the supine position, the pain relief device may be activated when the person changes to prone position.

As another example, pain definition for person A may be lack of movement of left leg. If this is triggered, pain relief techniques may be activated. Moreover, other physiological signals may also be used for description of pain during sleep. In particular, changes in the respiration, heart rate, skin temperature, and/or blood pressure may be indicative of painful periods.

The acquired sensor data relating to the user or the user's condition may therefore be used to control a sleep assistance device, which is a device for alleviating or reducing a user's pain or discomfort. The device may be controlled in accordance with a predicted pain measure or a future sleep disturbance event, so as to reduce the likelihood of sleep disturbance occurring.

Upon predicting a sleep disturbance, the desire is to prevent the disturbance from occurring. However, it may not be immediately clear what the best way to avoid the disturbance is (for example, which sleep assistance device from multiple devices to select, or the settings of the devices). Embodiments of the present invention may be considered as a control apparatus in which one or more sleep assistance devices are continuously tuned based on the feedback from the user (as determined from the sensor data), with minimal intervention during sleep desired.

The sleep assistance device may, for example, be a pain relief device or a position change device. Additionally or alternatively, the sleep assistance device may be a sound generation device, a temperature changing device, an air quality and air circulation changing device, and/or a medication providing device. A pain relief device may apply a stimulation to the individual to reduce pain. The stimulation may, for example, be an electrical stimulation, light stimulation and/or heat stimulation. The stimulation may increase blood flow to an area causing pain so as to reduce the pain. An example of such a pain relief device is the Philips BlueTouch pain relief patch. The BlueTouch device uses blue LED light to reduce pain by providing soothing warmth to an area of pain of a user, such as, for example, back pain. The blue LED light stimulates the production of nitric oxide (NO) in the body, which promotes the circulation. As a consequence, the supply of oxygen and nutrients to the muscle is improved and, at the same time, pain transmission is reduced. The muscles may relax and pain may be relieved. NO is said to have antioxidant, cytoprotective and antiinflammatory characteristics. It is thus able to protect muscles and nerves against damage and prevent further injuries. The Philips BlueTouch device may therefore reduce pain in a user, which, when applied during sleep, may prevent or reduce sleep disturbances. Although the Philips BlueTouch device is discussed here, any other device directed to pain relief may be used as a sleep assistance device. Such devices include, but are not limited to, nerve stimulation devices (for example, transcutaneous electrical nerve stimulation), spinal cord stimulation, implantable devices, medication release devices, and/or massaging devices.

Due to their nature, pain relief devices, such as the Philips BlueTouch device, may have limited application times. For example, the Philips BlueTouch device has a recommended application time of 15 to 30 minutes, depending on the mode of the device. The operation time of the device may therefore be taken into account when generating the control signal. For example, if the sleep assistance device is a pain relief device that has a maximum application time of 30 minutes, the control signal may be generated to ensure that the pain relief device is not used for longer than 30 minutes. If the pain relief device has reached its maximum application time, the processor may select another sleep assistance device from among a plurality of sleep assistance devices for pain relief.

The pain relief device may also be a medication dispenser. For example, Parkinson's disease patients have such a device that gradually releases medication. Such a device may therefore be controlled in accordance with the predicted pain measure of the user and/or a predicted sleep disturbance event. For example, the device may release medication depending on a predicted sleep disturbance event if needed.

A position change device is a device that causes the user to change position when sleeping, preferably with minimal disturbance to the user (for example, using haptic and/or audio stimulation). The position change device may cause the user to move to a position that may reduce pain or be less likely to result in pain or discomfort. For example, the position may be suited to improved circulation, or may be a position that does not aggravate an injury of the user.

An example of a position change device is the Philips SmartSleep Snoring Relief Band. The Snoring Relief Band is a device that is worn around the chest of the user and uses vibrations to cause the user to move position while sleeping. For example, the device delivers gentle vibrations to cause the user to move from a position in which they are lying on their back to a position in which they are lying on their side. The Snoring Relief Band is used to cause the user to move to a position in which they are less likely to snore. However, the technology of the device may also be used to prompt the user to move to a position that is likely to relieve pain or is less likely to cause pain or discomfort. Although the Philips SmartSleep Snoring Relief Band is discussed here, any other device directed to causing the user to move may be used as a sleep assistance device. Such devices include, but are not limited to, a smart watch that can trigger user movement by haptic or auditory stimuli, and/or a movement device integrated in bed, etc.

The sensor data may be acquired from any device or sensor which is capable of detecting appropriate metrics of the user that provide information on the user's sleep. For example, the sensor data may be electroencephalogram (EEG) data acquired from an EEG sensor or the sensor data may be movement data acquired from an accelerometer. EEG data provides information on the electrical activity of the user's brain and it is possible to determine information about the user's sleep from this information. For example, it is possible to determine the stage of sleep of the user. Furthermore, it is also possible to determine whether the user feels pain or discomfort and whether a sleep disturbance is likely.

Movement data may be acquired from a device such as an accelerometer to monitor movement of the user. The user is more likely to move in certain stages of sleep compared with other stages of sleep and so the stage of sleep may be estimated from the movement data. Furthermore, an increase in movement may be indicative of pain or discomfort of the user. Increased restlessness may also indicate an approaching sleep disturbance event. A sleep disturbance may therefore be predicted from the movement data.

The sensor data may comprise multiple types of data received from one or more sensor devices. For example, the sensor data may comprise both EEG data and movement data. The use of multiple data types may increase the accuracy of the sleep disturbance prediction. The data may be monitored in real-time so that a pain measure of the user may be assessed in real-time and appropriate control of one or more sleep assistance devices may be applied. Similarly, the real-time monitoring of the sensor data may enable prediction of a sleep disturbance event in a subsequent time period. Appropriate control of one or more sleep assistance devices may be applied in accordance with a predicted sleep disturbance event so as to reduce the likelihood of the sleep disturbance event occurring, thereby improving the user's sleep quality. That is, a future sleep disturbance event may be predicted using, for example, the interaction of the EEG, movement, and position data to minimize sleep disturbances. The control and usage of one or more sleep assistance devices occurs based on the predicted sleep disturbance (i.e. before disturbance happens) so as to provide intervention and decrease the likelihood of the disturbance happening.

An example of a sensor device for acquiring the sensor data and providing it to the apparatus is the Philips SmartSleep Deep Sleep Head Band. The Deep Sleep Head Band is worn by the user during sleep and collects EEG data using sensitive sensors. The Deep Sleep Head Band detects deep sleep in real-time based on the EEG data. The Deep Sleep Head Band can also provide acoustic stimulation to the user to enhance slow waves (slow wave activity) during sleep, thereby improving the quality of sleep. Thus, a sensor device for acquiring sensor data and a sleep assistance device may be the same device.

Monitoring and analysis of the sensor data, such as, for example, the user movement data and EEG data, in real-time therefore allows the user's pain level to be monitored and potential sleep disturbances to be predicted, and appropriate action may be taken to reduce the chance of the sleep disturbance happening. Additional data, such as further information on the user or their lifestyle (for example, age, gender, medical conditions, circadian rhythm, etc.) and/or information on the user's sleep environment (for example, temperature, noise, whether they share a bed, etc.) may also be used in the prediction of the pain level and sleep disturbance events. Further information on the user or their sleep environment may improve the accuracy of the analysis of the sensor data since such additional factors may have an effect on the user's sleep and sleep cycle. The additional information may also be used in the generation of the control signal to the sleep assistance device so that the control of the sleep assistance device may be further improved.

One or more sleep assistance devices may be activated or, if they are already active, their settings may be adjusted in accordance with the predicted pain measure and/or the predicted sleep disturbance event. The settings of the device(s) may be tailored according to the analyzed sensor data and the pain level and/or predicted sleep disturbance event. The sleep assistance devices may therefore be controlled to reduce the pain level of the user and/or to avoid the sleep disturbance event. Multiple sleep assistance devices may be used in cooperation to provide the most suitable pain relief to the user. The control of multiple devices together may be provided by the apparatus based on the analysis of the sleep data, as well as the analysis of any additional data.

If multiple sleep assistance devices are available, the apparatus may select which of the devices to control based on the received sensor data. Additional data, such as the user or environment information, may also be used in the determination. One or more appropriate devices from among a number of available devices may therefore be selected that are best suited to the user at that moment so as to effectively relieve pain and/or avoid a predicted sleep disturbance event. The settings of a selected device may be controlled with respect to the settings of other selected devices. The sleep assistance devices may be a pain relief device and a position change device which may be considered together to determine which device should and can be activated to provide the most appropriate relief to the user and the device settings to achieve the relief.

The sleep assistance devices may have operation restrictions or rules, which may be dependent on the use of other devices. For example, it may be preferable that two particular devices are not used at the same time, or it may be preferable that one device is used before another device. Considering the example of the pain relief device and the position change device being used in collaboration, it may be preferable that both devices are not activated simultaneously so as to increase the coverage during the night (because, for example, the pain relief device can be used only for limited time) and to minimize unintended disturbances (for example, the position change device can disturb the user, or an individual that the user shares a bed with). In some circumstances (for example, close to morning) both devices may be used simultaneously. The interaction between the pain relief device and the position change device may be explored and optimized to improve sleep quality and reduce sleep disturbances.

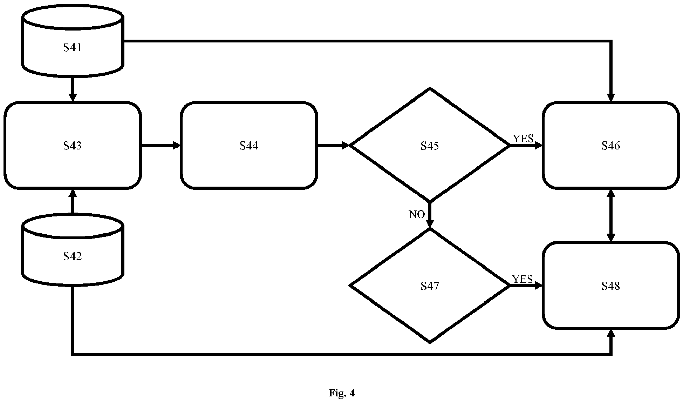

Fig. 4 shows a flow diagram of a control method according to an embodiment of an aspect of the invention. Movement data and EEG data is collected at step S41 and step S42, respectively. This data is used in real-time sleep data analysis at step S43. Next, at step S44 a pain measure is predicted and a sleep disturbance (such as, for example, a wake up event) is predicted using the analyzed sleep data and, from the predictions, it is determined whether to activate a sleep assistance device. Thus, at step S45, it is determined whether to activate the position change device, with the position change device settings adjusted at step S46 if the position change device is activated. If it is determined not to use the position change device, then a determination of whether to use the pain relief device is performed at step S47. The pain relief device setting are adjusted at step S48 if the pain relief device is activated. The adjustments of the position change device settings and the pain relief device settings are therefore made in accordance with the sleep data analysis and the resulting predicted pain measure and sleep disturbance prediction. As can be in Fig. 4, the movement data may also be utilized in the adjustment of the positon change device settings and the EEG data may similarly be utilized in the adjustment of the pain relief device settings. The settings of the position change device and/or the pain relief device may also be fed back to be used in the sleep data analysis (not shown).

Thus, future sleep disturbance events may be predicted ahead of time so that action may be taken to attempt to avoid the sleep disturbance events. The sleep disturbance events may be associated with the pain measure of the user.

In the example shown in Fig. 4, it is preferable for the position change device to be used ahead of the pain relief device. However, the pain relief device may be used ahead of the position change device and/or the devices may be used at the same time. The use of the devices may depend on a number of factors including the sensor data, the user data and/or the environment data.

In certain circumstances, the pain relief device may have a set usability time, which cannot be exceeded. Many different pain relief options may be available to use. The position changing device may not generally have such a usability limit. However, in a case in which the user shares a bed with another individual, a limit on the position change device may also be imposed so as to reduce the risk of disturbing the other individual. The apparatus may determine which device to use based on the real-time analysis of, for example, EEG and movement data to prevent or minimize a predicted sleep disturbance.

In an embodiment of an aspect, the first option is always to use position changing device. The sleep data continues to be monitored after the position change device has been activated/controlled and if it is determined (for example, from movement and EEG data) that the user is still restless after the position change, the data is re-evaluated and a new device usage decision may be made. The second option includes using position change or pain relief device, but it is again preferred to use the position change device ahead of the pain relief device. This can be implemented by having a stricter threshold for pain relief device usage (or a lower weight in case of classifier-like implementation). If there is a need for a new re-evaluation, both devices are equally likely to be selected. If the pain relief device has been used and has reached its usage limit, the position changing device is used.

The decision on which pain relief device settings to use may be driven by the nature of the discomfort or pain, as well as the allowed usage time limit, and the position and estimated wake-up time of the user. Other sleep characteristics (for example, time in bed, duration of deep sleep, etc.) or lifestyle characteristics (for example, stress, activity, nutrition) characteristics may also be used. Multiple devices may be used together. For example, if a pain relief device provides localized stimulation to a specific area of the user, the position change device may be used prior to the pain relief device to move the user to an optimal position for the pain relief device. The pain relief device may be provided as part of a bed, rather than as a wearable sensor, and so it may be necessary to cause the user to move to the pain relief device using the position change device prior to using the pain relief device.

Information from the sleep assistance devices may be fed back to the apparatus to be used in the determination of the sleep disturbance event and/or the generation of the control signal for one or more sleep assistance devices, which may be the same or different devices from those providing feedback. Alternatively or additionally, the generated control signal may be an updated control signal for the sleep assistance devices providing the feedback. The kind of information that is provided by the sleep assistance devices may depend on the intelligence and features of the devices. In the simplest case, information on the usage time, the usage duration and the intensity settings may be shared. For sleep assistance devices that are able to provide localized and targeted solutions, then the location of pain relief, and the user position change information may also be shared.

The apparatus may learn the best position for sleep and the best position to provide pain relief support, as well as the sleep assistance device settings for the user over time. The best position for sleep and the best position for providing pain relief may be different. Therefore, according to an embodiment of an aspect, the position change device may bring the user to the best position for application of the pain relief device, instead of aiming to minimize the user pain by position change. That is, multiple devices can be controlled in cooperation.

An initialization of the apparatus may be performed daily prior to the user falling to sleep. In the initialization, the user's position is measured before falling asleep since it is assumed that the user will consciously try to assume a position in which they are comfortable and do not feel pain or discomfort, or feel the minimum amount of pain or discomfort. It may be preferable to perform the initialization daily because the nature of pain may evolve over time and so the preferred position of the user may change accordingly. Of course, the initialization can be performed at more or less frequent intervals, for example, once every three days, weekly or fortnightly. The decision of the frequency of the initializations may be learned by the apparatus from the user data over time. For example, if the position at which the user feels minimal pain or discomfort varies most nights, then it may be beneficial to perform the initialization frequently, for example, nightly so that the apparatus can acquire the optimum sleep position for the night. Conversely, if the user has consistently felt a minimal amount of pain in a given position for a number of nights, the apparatus may use this position as the preferred position of the user and initialization may not be necessary. Performing regular (for example, daily) initializations will enable the apparatus to track the pain or comfort level change of the user over time. This may be calculated as a function of sleep duration, and device usage time (for example, sleep duration / (pain relief device usage time + position change device usage time)). This is just one particular way for calculating the score. In general, different methods may be devices from using sleep signals (e.g. EEG or actigraphy) and sleep assistance devices parameters. For this particular equation, if it is observed that the user is able to have longer durations of sleep without using any external help, this is positive and desired indication.

A number of methods may be used for predicting a sleep disturbance event.

### Method 1: Sleep stage specific sleep disturbance prediction

The main idea of the first method is that every sleep stage is defined by specific brain wave (EEG) features which are well established. Moreover, the brain wave features may be personalized to the user by monitoring the user and their EEG data for a number of nights.

The first method comprises the following steps:
- Step 1: Detect current sleep stage: use historical data, or look up table to quantitatively describe the sleep stage, for example, in terms of EEG signal frequency range and amplitude, user movement, heart rate, heart rate variability, respiration rate, etc. features.
- Step 2: Continuously check if the calculated features (for example, frequency, amplitude) are within the expected range. If not (for a specified amount of time), predict a sleep disturbance event.

The first method therefore compares the sleep data with known data and predicts a sleep disturbance event if the sleep data deviates from the expected data since a deviation from the expected pattern may be indicative of approaching disturbance. That is, since the sleep stages come in order and every sleep stage has relatively well defined features, these features may be monitored to determine disturbance. In this case, the disturbance will be predicted based on the trend of the features. In other words, if, for example, the trend indicates a change which is not in line with what is expected, then this can be considered as a disturbance indicator.

For example, during deep sleep, if the frequency of the EEG waves is increasing, or if the amplitude of the delta waves is decreasing, these can be taken as disturbance indicators. Note, however, that a decrease in frequency, and an increase in delta amplitude may not be considered as a disturbance because it is in line with the deep sleep changes.

### Method 2: Using machine learning to predict sleep disturbance in a linear manner

While the first method uses EEG as the main data, the second method uses additional parameters in the prediction, such as, for example, movement, respirations, heart rate, skin temperature, blood pressure, and some other physiological parameters. The machine learning method may be the preferred method for pain prediction.

The second method comprises the following steps:
- Step 1: Collect user data for a period of time, such as, for example, one week, and use a short time window (for example, 30 seconds) to train a binary classifier (awake or not-awake), for linear prediction (for example, predict the following window features, from the previous windows).
- Step 2: Observe the output of the classifier to predict a disturbance (for example, a disturbance is predicted if the likelihood of 'awake' exceeds 90%).
- Classifiers such as convolutional neural network (CNN), support vector machine (SVM), random forest and K-means clustering may be used.

Thus, the approach of the second method is to train a binary predictor that is able to assign a likelihood score for the next time period, such as, for example, 30 seconds or 60 seconds based on the past samples. The aim here is to determine the likelihood of the user waking, and to take action (i.e. control sleep assistance devices) in accordance with the likelihood.

If, during application of a pain relief device, the likelihood for the user waking up is higher than a threshold, use of the device should be stopped so that the user is not disturbed. If, during sleep, the likelihood of the user waking up is higher than a threshold and pain is determined/predicted (from the changes in the physiological signals), then one or more sleep assistance devices, such as pain relief devices, may be activated and the settings of the pain relief devices set accordingly.

### Method 3: Use machine learning to predict sleep activity in a global manner, for the whole duration of the night

The third method is similar to the second method yet longer term predictions (for example, predictions for the next hour, rather than the next minute) of the sleep stages and wake up periods are performed.

The third method comprises the following steps:
- Step 1: Collect user data for a longer time period (for example, one month). Train an algorithm (for example, CNN) to predict the wake up times throughout the night, as a function of going to bed time, and first sleep cycle (onset-I-II-III-IV-REM).
- Step 2: Only use the devices outside of the predicted wake-up periods.

The third method enables the application of pain relief to be activated during deep sleep stages (stages 3 and 4) prior to the predicted wake-up period. The idea here is to find the optimal time to apply the pain relief by better aligning the application to be during certain sleep stages.

### Method 4: A combination of the first to third methods

An alternative approach may be to use the first, second and third methods in combination. For example, the methods may be applied sequentially, such that method 3 is applied first, followed by method 2 and then method 1.

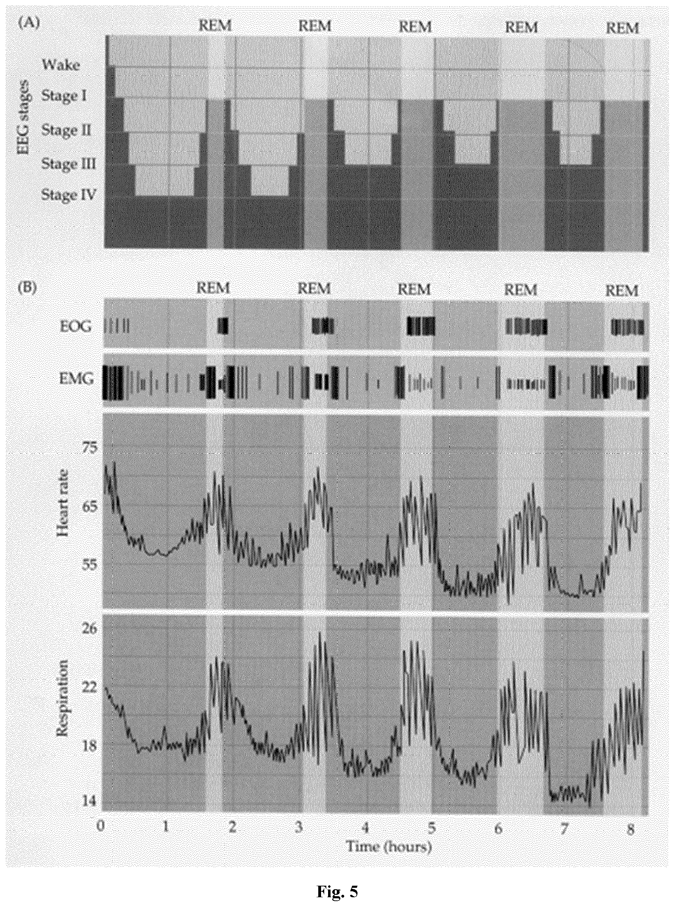

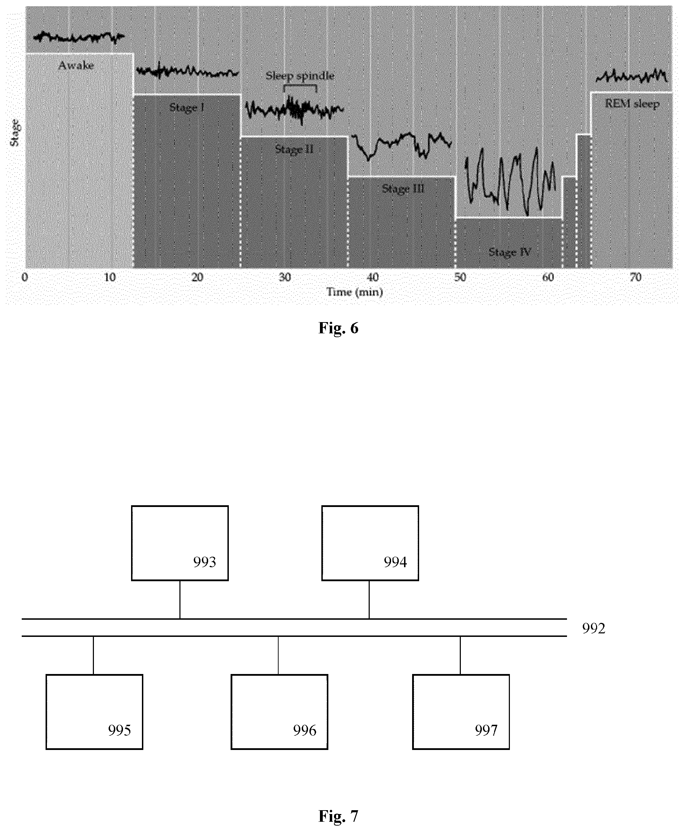

Fig. 5 shows a graph of the expected physiological readings of a user in the different stages of sleep, which may be used in the sleep disturbance prediction methods, as discussed above. The graph shows the expected physiological data against sleep time and the stages of sleep, and includes heart rate and respiration. Fig. 6 shows a graph of sleep stages against time in a standard sleep cycle. Table 1 below lists the expected EEG data (frequency and amplitude) for each stage of sleep.

**Table 1: Expected EEG data for sleep stages**

| Stage of Sleep | Expected Data |
|---|---|
| Awake/REM: Beta activity | EEG freq. 15-50Hz |
| | EEG amplitude: 30µV |
| Stage I: Theta waves | EEG freq. 4-8Hz |
| | EEG amplitude: 50-100 µV |
| Stage II: Spindles | EEG freq. 10-15Hz |
| | EEG amplitude: 50-150µV |
| Stage III | EEG freq. 2-4Hz |
| | EEG amplitude: 100-150µV |
| Stage IV: Delta waves | EEG freq. 0.5-2Hz |
| | EEG amplitude: 100-200µV |

Embodiments of aspects may therefore provide an apparatus and method for alleviating a user's pain during sleep in which a control signal may be generated and transmitted to a sleep assistance device in accordance with a predicted pain measure of the user. Sleep disturbances and/or interruptions may therefore be reduced or avoided and the quality of sleep of the individual may be improved. Furthermore, the operation of the sleep assistance device may be controlled, such that the effectiveness of the device may be maximized and the pain or discomfort of the individual may be reduced.

Fig. 7 is a block diagram of a computing device, such as a server incorporating resources suitable for sensor data processing, which may embody the present invention, and which may be used to implement some or all of the steps of a method embodying the present invention, and perform some or all of the tasks of an apparatus of an embodiment. For example, the computing device of Fig. 7 may be used to implement all, or only some, of steps S21 to S24 of the method illustrated in Fig. 2, to perform all, or only some, of the tasks of the apparatus shown in Fig. 1 to perform all, or only some, of the tasks of receiver 11, processor 12 and/or transmitter 13. The computing device comprises a processor 993, and memory 994, and to implement all, or only some, of steps S41 to S48 of the process illustrated in Fig. 4. Optionally, the computing device also includes a network interface 997 for communication with other computing devices, for example with other computing devices of invention embodiments.

For example, an embodiment may be composed of a network of such computing devices. Optionally, the computing device may also include one or more input mechanisms 996 such as a keyboard and mouse for the user to input any of, for example, user information or preference settings of the sleep assistance device, and a display unit 995 such as one or more monitors. The display unit may show a representation of data stored by the computing device for instance, representations of the determined pain level, predicted sleep disturbance events and sleep assistance device settings. The display unit 995 may also display a cursor and dialogue boxes and screens enabling interaction between a user and the programs and data stored on the computing device. The input mechanisms 996 may enable a user to input data and instructions to the computing device. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions described here and in the claims. The memory 994 stores data being read and written by the processor 993, such as the inputs (such as, for example, the received sensor data), interim results (such as, for example, analysis of the sensor data and determined pain level) and results of the processes referred to above (such as, for example, the control signal to be transmitted). As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 995 may display a representation of data stored by the computing device and may also display a cursor and dialog boxes and screens enabling interaction between a user and the programs and data stored on the computing device. The input mechanisms 996 may enable a user to input data and instructions to the computing device. The display unit 995 and input mechanisms 996 may form the output 26.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc. may be included in the computing device.

Methods embodying the present invention may be carried out on a computing device such as that illustrated in Fig. 7. Such a computing device need not have every component illustrated in Fig. 7 and may be composed of a subset of those components. A method embodying the present invention may be carried out by a single computing device in communication with one or more data storage servers via a network. The computing device may be a data storage itself storing the input content before and after processing and thus for example, the dialogue and/or trained model.

A method embodying the present invention may be carried out by a plurality of computing devices operating in cooperation with one another. One or more of the plurality of computing devices may be a data storage server storing at least a portion of the data.

Other hardware arrangements, such as laptops, iPads and tablet PCs in general could alternatively be provided. The software for carrying out the method of invention embodiments as well as input content, and any other file required may be downloaded, for example over a network such as the internet, or using removable media. Any dialogue or trained model may be stored, written onto removable media or downloaded over a network.

The invention embodiments may be applied to any field in which a user is monitored during sleep and measures are taken to reduce the user's pain and avoid sleep disturbance. The invention embodiments may preferably applied to the medical and healthcare field.

Artificial neural networks are widely employed to perform pattern matching and diagnostic procedures, using so-called "machine learning". A typical structure of an artificial neural network is a three-layer structure, having an input layer at which observations are input to the network, a hidden or processing layer, at which further processing operations are carried out on information received from the input layer, and an output layer, at which an output signal is generated based on information received from the processing layer. The precise structure of the artificial neural network is not limited, neither are the specific functions of the layers.

A suitable neural network system may include a training processor which utilizes test data and annotated data to generate a trained model for an AI system, which trained model is accessible by an AI system. Detection is performed with reference to a similarity value computation processor.

Such a system comprises a hardware architecture such as that illustrated in Fig. 7 (described above), which may be used for training a model using the sensor data. In particular, processor 993 may perform the processing instructions for training the model, determining the pain level of the user and/or predicting a sleep disturbance event and providing the response and the associated processing. One or more storage units (memories) 994 store the processing instructions, the trained model query response and the dialogue.

Variations to the disclosed embodiments may be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

The above-described embodiments of the present invention may advantageously be used independently of any other of the embodiments or in any feasible combination with one or more others of the embodiments.

## Claims

1. An apparatus (10) to control a sleep assistance device (30) for alleviating a user's pain during sleep, the apparatus (10) comprising:
a receiver (11) configured to receive sensor data (14) associated with the user;
a processor (12) configured to analyze the sensor data (14) to predict a pain measure of the user, and to generate a control signal (15) for the sleep assistance device (30) in accordance with the predicted pain measure; and
a transmitter (13) configured to transmit the control signal (15) to the sleep assistance device (30) for alleviating pain.

2. The apparatus (10) according to claim 1, wherein the processor (12) is configured to:
compare the sensor data (14) with known data; and
predict the pain measure of the user based on the comparison of the sensor data (14) and the known data.

3. The apparatus (10) according to claim 2, wherein the processor (12) is configured to:
analyze the sensor data (14) to determine a stage of sleep of the user;
predict the pain measure of the user based on the sensor data (14) and the stage of sleep of the user.

4. The apparatus (10) according any preceding claim, wherein
the processor (12) is configured to analyze the sensor data (14) to predict a sleep disturbance event in a predetermined time period; and
the transmitter (13) is configured to generate the control signal (15) for the sleep assistance device (30) in accordance with the predicted sleep disturbance event so as to minimize the possibility of occurrence of the predicted sleep disturbance event.

5. The apparatus (10) according to claim 4, wherein the processor (12) is configured to:
analyze the sensor data (14) to determine a stage of sleep of the user;
compare the sensor data (14) with expected data for the stage of sleep; and
predict the sleep disturbance event based on the comparison of the sensor data (14) and the expected data.

6. The apparatus (10) according to claim 4 or 5, wherein the processor (12) is configured to:
store the sensor data (14) in a memory (40) associated with the apparatus (10);
train a classifier using the sensor data (14) stored in the memory (40); and
predict the sleep disturbance event in accordance with a likelihood score of the classifier.

7. The apparatus (10) according to any preceding claim, wherein
during a sleep onset period prior to the user falling asleep , the receiver (11) is configured to receive initialization information;
the initialization information comprises data indicative of one or more of: a position of the user, and physiological signals of the user; and
the processor (12) is configured to predict the pain measure of the user based on the sensor data (14) and the initialization information.

8. The apparatus (10) according to any preceding claim, wherein
the receiver (11) is configured to receive user data;
the processor (12) is configured to predict the pain measure of the user based on the sensor data (14) and the user data; and
the user data comprises one or more of:
the age of the user;
medical history of the user;
medication prescribed to the user;
medication taken by the user;
a medical diagnosis of the user;
lifestyle information of the user;
sleeping preferences of the user;
sleep environment of the user;
a location of the user;
the gender of the user;
the ethnicity of the user;
physical activity of the user;
information on the user's preferred operating settings of the sleep assistance device (30);
subjective input of the user; and
an occupation of the user.

9. The apparatus (10) according to any preceding claim, wherein
the sleep assistance device (30) is one of a plurality of sleep assistance devices; and
the processor (12) is configured to select the sleep assistance device (30) from among the plurality of sleep assistance devices by analyzing the sensor data (14).

10. The apparatus (10) according to any preceding claim, wherein
the processor (12) is configured to determine a setting instruction for the sleep assistance device (30) in accordance with the predicted pain measure of the user; and
the control signal (15) comprises the setting instruction for the sleep assistance device (30).

11. The apparatus (10) according to any preceding claim, wherein
the receiver (11) is configured to receive feedback data (16) from the sleep assistance device (30), the feedback data (16) comprising one or more of:
present setting instruction of the sleep assistance device (30);
a duration of operation of the sleep assistance device (30);
status information of the sleep assistance device (30); and
operating restrictions of the sleep assistance device (30); and
the processor (12) is configured to generate the control signal (15) in accordance with the feedback data (16).

12. The apparatus (10) according to claim 11, wherein the processor (12) is configured to:
store the feedback information in a memory (40) associated with the apparatus (10) in accordance with the sensor data (14) received with the feedback information;
analyze the stored feedback information and sensor data (14) to determine an optimum mode of operation of the sleep assistance device (30) for the user; and
generate the control signal (15) in accordance with the optimum mode of operation of the sleep assistance device (30).

13. The apparatus (10) according to any preceding claim, wherein the sensor data (14) is one or more of:
electroencephalogram, EEG, data;
movement data;
image data;
respiration data;
heart data;
electrodermal data;
blood pressure data;
audio data; and
temperature data.

14. A method of controlling a sleep assistance device (30) for alleviating a user's pain during sleep, the method comprising:
receiving (S21) sensor data (14) associated with the user;
analyzing (S22) the sensor data (14) to predict a pain measure of the user;
generating (S23) a control signal (15) for the sleep assistance device (30) in accordance with the predicted pain measure; and
transmitting (S24) the control signal (15) to the sleep assistance device (30) for alleviating pain.

15. A computer program which, when executed on a computing system, carries out a method of controlling a sleep assistance device (30) for alleviating a user's pain during sleep, the method comprising:
receiving (S21) sensor data (14) associated with the user;
analyzing (S22) the sensor data (14) to predict a pain measure of the user;
generating (S23) a control signal (15) for the sleep assistance device (30) in accordance with the predicted pain measure; and
transmitting (S24) the control signal (15) to the sleep assistance device (30) for alleviating pain.
